# EUROPÄISCHE PATENTANMELDUNG

(11) **EP 1 623 672 A1**
(43) Veröffentlichungstag der Anmeldung: **08.02.2006**
(21) Anmeldenummer: 04018504.3
(22) Anmeldetag: 04.08.2004
(51) Int. Cl.: A61B 6/06, G21K 1/04, H05G 1/52

(54) **Röntgenvorrichtung, insbesondere für ein Mammographie-Röntgengerät**

(71) Anmelder: SIEMENS AKTIENGESELLSCHAFT, 80333 München (DE)
(72) Erfinder: Freudenberger, Jörg, Dr., 90542 Eckental (DE); Schardt, Peter, Dr., 91315 Höchstadt A.D. Aisch (DE)

(57) **Zusammenfassung**

Zur Verbesserung der Bildauflösung ist bei einer Röntgenvorrichtung mit einer Röntgenröhre (1) zur Erzeugung eines von einem Brennfleck (7) einer um eine Drehachse (3) drehbaren Drehanode (2) ausgehenden Röntgenstrahles und mit einer schlitzförmigen Blende (4) zur Erzeugung eines aus dem Röntgenstrahl einblendbaren, scannerartig über einen Untersuchungsbereich (8) bewegbaren, fächerförmigen Strahlenbündels (5) vorgesehen, dass das fächerförmige Strahlenbündel (5) im wesentlichen in Richtung der Drehachse (3) der Drehanode (2) über den Untersuchungsbereich (8) bewegbar ist und die Röntgenröhre (1) derart um den Brennfleck (7) kippbar ist, dass das fächerförmige Röntgenstrahlenbündel (5) bei der Bewegung über den Untersuchungsbereich (8) im Bereich höchster Bildauflösung bzw. höchster Bildschärfe liegt.

## Beschreibung

Die Erfindung bezieht sich auf eine Röntgenvorrichtung, insbesondere für ein Mammographie-Röntgengerät, gemäß dem Patentanspruch 1.

In der Röntgenbildgebung werden die Abbildungseigenschaften durch die Streustrahlung eines von einer Anode, zum Beispiel einer Drehanode, erzeugten Röntgenstrahles und dadurch bedingte Unschärfe negativ beeinflusst. Zur Vermeidung der Streustrahlung kommen neben Streustrahlenrastern Scanverfahren zum Einsatz, bei denen mittels einer schlitzförmigen Blende ein fächerförmiges Röntgenstrahlenbündel aus dem Röntgenstrahl ausgeschnitten und die Blende mit dem jeweiligen Röntgenstrahlenbündel über das abzubildende Objekt rasterartig bewegt wird. Zur Verminderung der Streustrahlung ist es weiterhin bekannt, eine weitere Schlitzblende zwischen dem Objekt und einem Detektor zur Umwandlung von Röntgenstrahlung in ein Bildsignal vorzusehen oder den Detektor in zeilenartige Bereiche aufzuteilen und lediglich den vom Röntgenstrahlenbündel jeweils überstrichenen Bereich abzubilden. Es lässt sich jedoch nicht vermeiden, dass mittels der Bewegung der Blende Bereiche des Röntgenstrahles eingeblendet werden, die eine unterschiedliche Bildauflösung erzeugen, so dass die entstehende Abbildung unterschiedliche Bildschärfen aufweist.

Aus der US 4,928,297 ist eine Röntgenvorrichtung für ein Scanverfahren bekannt, bei der eine Röntgenröhre, eine schlitzförmige Blende und ein zeilenartiger Detektor auf einer Linie angeordnet und gemeinsam an einem Schaft befestigt und dann diese Baueinheit um eine oberhalb der Röntgenröhre die Linie schneidende Achse drehbar angeordnet ist; dadurch ist die notwendige Synchronisation der mechanischen Bewegungen besonders einfach.

Es ist Aufgabe der vorliegenden Erfindung, eine Röntgenvorrichtung anzugeben, bei der auf aufwandsarme Weise eine Verbesserung der Qualität von Röntgenabbildungen, insbesondere hinsichtlich einer über den gesamten Bereich scharfen Abbildung, erzielt werden kann.

Diese Aufgabe wird erfindungsgemäß gelöst durch eine Röntgenvorrichtung gemäß Patentanspruch 1 bzw. durch ein Mammographie-Röntgengerät mit einer solchen Röntgenvorrichtung. Durch das erfindungsgemäße Kippen der Röntgenröhre kann mit einfachen Mittels erreicht werden, dass das Röntgenstrahlenbündel im Bereich höchster Bildauflösung bzw. Bildschärfe des Röntgenstrahles, welcher auf der der Drehanode zugewandten Seite des Röntgenstrahles liegt, über den Untersuchungsbereich bewegt wird und somit insgesamt eine gleichmäßige und hohe Bildschärfe bzw. hohe Bildauflösung über den Untersuchungsbereich gewährleistet ist.

Die Abbildungsschärfe ist dadurch noch weiter verbesserbar, dass zusammen mit dem Kippen der Röntgenröhre die schlitzförmige Blende im Wesentlichen in Richtung der Drehachse der Drehanode und im Wesentlichen synchron zur Bewegung des fächerförmigen Röntgenstrahlbündels über den Untersuchungsbereich bewegbar ist; dadurch bleibt gewährleistet, dass jeweils der Bereich höchster Bildauflösung des Röntgenstrahlenbündels durch die schlitzförmige Blende eingeblendet ist.

In vorteilhafter Weise ist der Einsatz der erfindungsgemäßen Röntgenvorrichtung dadurch erweiterbar, dass die schlitzförmige Blende im Sinne eines den gesamten Untersuchungsbereich überdeckenden Schlitzes, d.h. im Vollfeld-Bereich, einstellbar ist. Dadurch ist die Röntgenvorrichtung auch für jede Art der Vollfeld-Untersuchung nutzbar; die Vollfelduntersuchung lässt sich durch die erfindungsgemäße Röntgenvorrichtung dadurch noch weiter verbessern, dass die schlitzförmige Blende im Sinne eines den gesamten Untersuchungsbereich überdeckenden Schlitzes verstellbar ausgebildet und das Röntgenstrahlenbündel mit seinem Bereich höchster Bildauflösung innerhalb dieses Schlitzes allein durch das Kippen der Röntgenröhre auf einen definierten Bereich des Untersuchungsbereiches einstellbar ist. Das Kippen der Röntgenröhre, das zu einer effektiven Verminderung des Anodenneigungswinkels der Drehanode führt, führt auch für eine Vollfeld-Untersuchung zu einer Verringerung der Bildschärfenunterschiede über dem Untersuchungsbereich und damit zu einer insgesamt schärferen Abbildung. Zusätzlich kann durch das Kippen der Röntgenröhre der Bereich mit der höchsten Bildschärfe passend auf das zu untersuchende Objekt eingestellt werden, so dass eine vollständige Nutzung dieses Bereichs möglich ist.

Nach einer weiteren Ausgestaltung der Erfindung ist der Brennfleckes in radialer Richtung der Drehanode vergrößerbar. Dadurch ist die Röntgenleistung durch die höhere Brennfleckfläche ohne Verlust an Bildschärfe steigerbar, da zur Nachregelung der Bildschärfe die Röhre erfindungsgemäß gekippt wird. Der Vorteil einer Steigerung der Röntgenleistung ist eine Verringerung der Scanzeit. Die dadurch bewirkte Verkürzung der Untersuchungszeit ist für einen mit der Röntgenvorrichtung zu untersuchenden Patienten erstrebenswert, da er während der Röntgenaufnahme nicht bewegt werden darf, was vor allem bei der Mammographie für den Patienten sehr schmerzhaft sein kann.

Zur variablen Einstellung und damit nach Bedarf wählbaren Vergrößerung des Brennfleckes sind bei einer erfindungsgemäßen Röntgenvorrichtung mit einer Kathode zur Erzeugung eines Elektronenstrahls in vorteilhafter Weise mindestens zwei Emitter, insbesondere unterschiedlicher Ausprägung zur Erzeugung eines Brennfleckes entsprechender Abmessungen, vorgesehen. Zweckmäßigerweise weist die Kathode mindestens einen Emitter mit mindestens einem Zwischenabgriff zur separaten Ansteuerung von Teilbereichen des Emitters auf. Durch das Zuschalten von Teilbereichen des Emitters sind die Abmessungen des Brennfleckes dadurch steigerbar. Es können auch mehrere Emitter mit mehreren Zwischenabgriffen vorgesehen sein, um weitere Einstellmöglichkeiten der Abmessungen des Brennfleckes vorzusehen.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert; es zeigen:
- FIG 1: eine bekannte Röntgenvorrichtung zur Erzeugung eines scannerartig bewegbaren Röntgenstrahlenbündels;
- FIG 2: eine erfindungsgemäße Röntgenvorrichtung in perspektivischer Ansicht in einer ersten Kippstellung;
- FIG 3: die erfindungsgemäße Röntgenvorrichtung gemäß FIG 2 in Seitenansicht;
- FIG 4: die erfindungsgemäße Röntgenvorrichtung in perspektivischer Ansicht gemäß FIG 2 in einer zweiten Kippstellung;
- FIG 5: die erfindungsgemäße Röntgenvorrichtung gemäß FIG 4 in Seitenansicht;
- FIG 6: die erfindungsgemäße Röntgenvorrichtung in perspektivischer Ansicht in einer dritten Kippstellung;
- FIG 7: die erfindungsgemäße Röntgenvorrichtung gemäß FIG 6 in Seitenansicht;
- FIG 8: die erfindungsgemäße Röntgenvorrichtung mit einer im Sinne einer Vollfeldaufnahme verstellten Blende;
- FIG 9: einen Ausschnitt aus der Röntgenvorrichtung gemäß FIG 2 mit einer Kathode;
- FIG 10: einen Ausschnitt aus der Kathode gemäß FIG 9;
- FIG 11: eine alternative Kathode gemäß FIG 9;
- FIG 12: die erfindungsgemäße Röntgenvorrichtung in Seitenansicht mit zusätzlicher erster Drehachse;
- FIG 13: eine Frontansicht auf die erfindungsgemäße Röntgenvorrichtung gemäß FIG 12;
- FIG 14: die erfindungsgemäße Röntgenvorrichtung in Seitenansicht mit zusätzlicher zweiter Drehachse;
- FIG 15: eine Frontansicht auf die erfindungsgemäße Röntgenvorrichtung gemäß FIG 14;
- FIG 16: die erfindungsgemäße Röntgenvorrichtung in Seitenansicht mit einer zusätzlichen detektornahen Blen-de;
- FIG 17: einen Detektor aus parallelen Detektorzeilen;
- FIG 18: die erfindungsgemäße Röntgenvorrichtung in Seitenansicht mit einzeln aktivierbaren Detektorzeilen;
- FIG 19: die erfindungsgemäße Röntgenvorrichtung in stirnseitiger Draufsicht mit einem mittig des Öffnungswinkels des Röntgenstrahlenbündels positionierten Brennfleck.

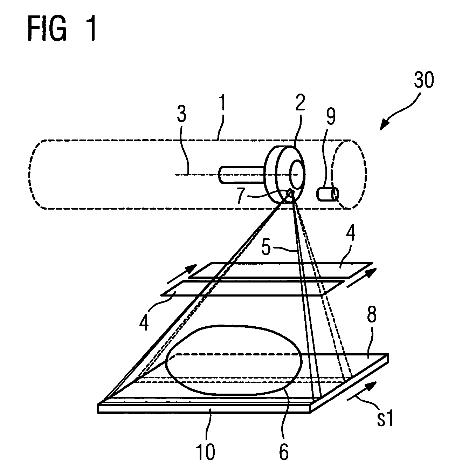

FIG 1 zeigt eine allgemein bekannte Röntgenvorrichtung 30 zur Abbildung eines Objektes 6 mit einer schlitzförmigen Blende 4 zur Erzeugung eines aus einem Röntgenstrahl einblendbaren, scannerartig über einen Untersuchungsbereich 8 bewegbaren, fächerförmigen Röntgenstrahlenbündels 5. Die Röntgenvorrichtung 30 enthält eine Röntgenröhre 1 mit einer um eine Drehachse 3 rotierbaren Drehanode 2 sowie einen flächenhaften, den gesamten Untersuchungsbereich des Objektes 6 erfassenden Detektor 10. Die Längserstreckung des fächerförmigen Röntgenstrahlenbündels 5 ist dabei im Wesentlichen parallel zur Drehachse 3 der Drehanode 2, die Scanrichtung über den Untersuchungsbereich liegt im Wesentlichen senkrecht zur Ebene des fächerförmigen Röntgenstrahlbündels. Das Scannen erfolgt durch die Bewegung der schlitzförmigen Blende 4 in Pfeilrichtung s1.

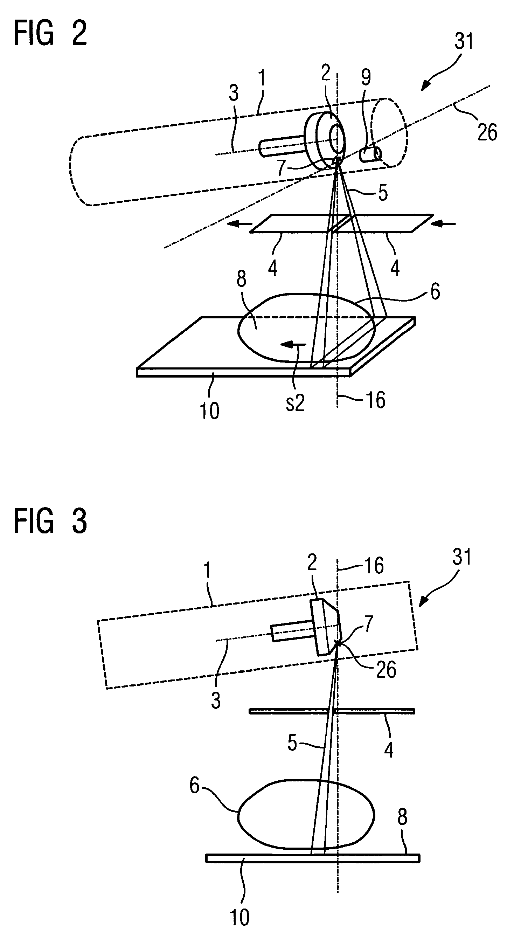

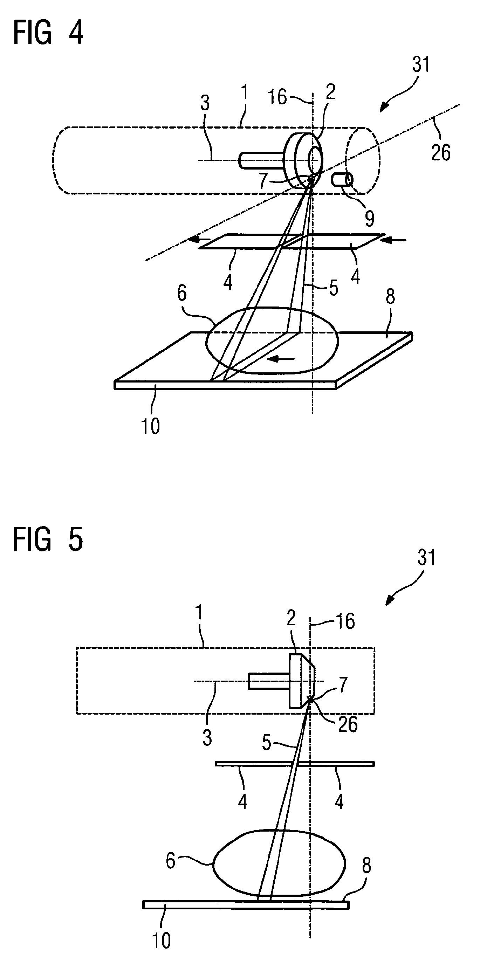

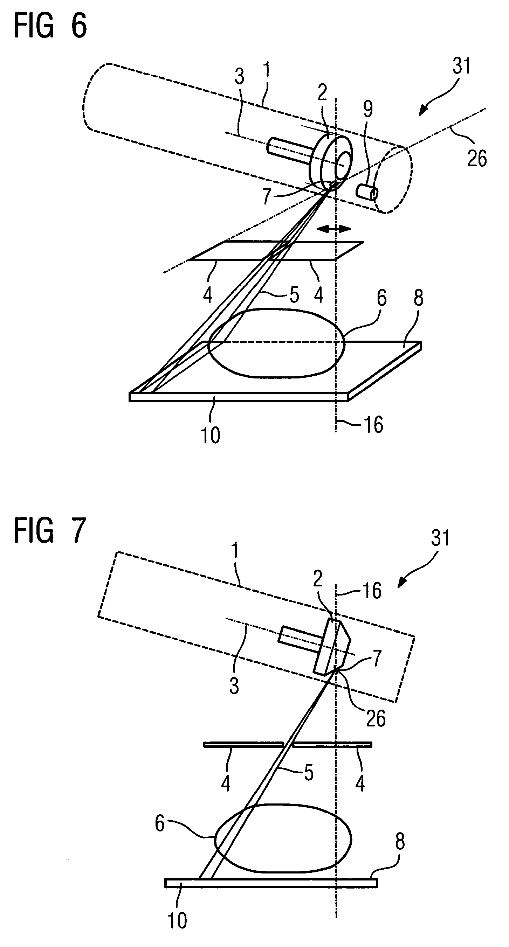

FIG 2 bis FIG 7 zeigen jeweils eine erfindungsgemäße Röntgenvorrichtung 31 zum Scannen eines Objektes 6 auf einem Untersuchungsbereich 8 in verschiedenen Kippstellungen der Röntgenröhre 1. Das fächerförmige Röntgenstrahlenbündel 5, welches mittels der schlitzförmigen Blende 4 eingeblendet wird, ist im Wesentlichen - wie anhand des Pfeils s2 auf dem Detektor 10 in FIG 2, 4 und 6 angedeutet - in Richtung der Drehachse 3 der Drehanode 2 und senkrecht zur Ebene des fächerförmigen Röntgenstrahlenbündels 5 über den Untersuchungsbereich 8 bewegbar. FIG 2 und 3 zeigen eine erste Kippstellung der Röntgenröhre 2. Mittels der schlitzförmigen Blende 4 wird ein Röntgenstrahlenbündel 5 eingeblendet, welches im Bereich der größten Bildschärfe der Drehanode 2 mit entsprechendem Anodenneigungswinkel, der Drehanode 2 zugewandt, liegt. Durch das Kippen der Röntgenröhre 1 um eine durch den Brennfleck 7 parallel zur Oberfläche des Untersuchungsbereiches 8 und senkrecht auf einer ersten Achse 16 verlaufenden Kippachse 26 wird das fächerförmige Röntgenstrahlenbündel 5 scannerartig über den Untersuchungsbereich 8 bewegt. Die Blende 4 wird dabei derartig mitbewegt, d.h. synchronisiert, dass dasselbe Röntgenstrahlenbündel 5 eingeblendet bleibt. FIG 4 und 5 zeigen eine zweite und FIG 6 und 7 eine dritte Kippstellung der Röntgenröhre 1.

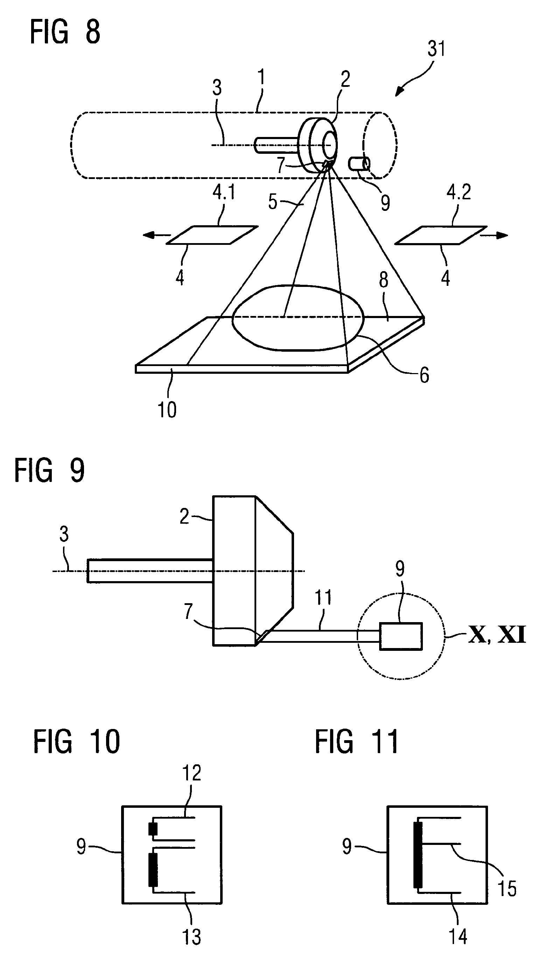

FIG 8 zeigt eine erfindungsgemäße Röntgenvorrichtung 31 mit einer im Sinne eines den gesamten Untersuchungsbereich überdeckenden Schlitzes ausgebildeten schlitzförmigen Blende 4. Die Blende 4 weist in vorteilhafter Weise zwei im Sinne einer Schlitzverstellung gegeneinander und /oder zueinander bewegbare, den Schlitz begrenzende Blendenbacken 4.1 und 4.2 auf; dadurch ist die erfindungsgemäße Röntgenvorrichtung auf einfache Weise auch im Vollfeldmodus einsetzbar. Dies ist vorteilhaft, da durch Vergleiche zwischen einer Abbildung mit Streustrahlenunterdrückung und ohne Streustrahlenunterdrückung desselben Objektes mit der gleichen Röntgenvorrichtung wichtige Informationen gewonnenen werden können. Außerdem ist durch den Vollfeldmodus die Möglichkeit von bildgebenden Verfahren gegeben, welche große Gesichtsfelder benötigen. Hierzu gehören insbesondere Biopsien. Das erfindungsgemäße Kippen der Röntgenröhre 1 bei aus dem Röntgenstrahl verfahrener Blende 4 ermöglicht dabei im Vollfeldmodus die Einstellung des Bereiches höchster Bildauflösung des Röntgenstrahlenbündels 5 auf jeden für die jeweilige Aufnahme durch die Kippung angesteuerten Teil des Untersuchungsbereiches.

Es ist außerdem möglich, nur eine einzelne Blendenbacke 4.1 bzw. 4.2 aus dem Röntgenstrahl zu verfahren und die andere zur Ausblendung von Teilbereichen des Röntgenstrahles zu verwenden. Die Blendenbacken 4.1; 4.2 können auch einzeln oder gemeinsam mit der Röntgenröhre 1 zu einer Einheit verbunden werden, so dass sie bei einer Kippung mitgekippt werden.

FIG 9 zeigt einen Ausschnitt aus einer erfindungsgemäßen Röntgenvorrichtung 31 mit einer Drehanode 2 und einer Kathode 9 zur Erzeugung eines Elektronenstrahles 11, welcher durch seine Auftrefffläche auf der Drehanode 2 die Größe des Brennflecks 7 bestimmt. In vorteilhafter Weise weist die Kathode 9 - wie in FIG 10 dargestellt - mindestens zwei Emitter 12, 13, insbesondere unterschiedlicher Ausprägung zur Erzeugung eines Brennfleckes 7 entsprechender Abmessungen, auf. Dadurch wird die Auswahl zwischen mindestens zwei unterschiedlich großen Brennflecken möglich. Durch die Wahl des größeren Brennfleckes gegenüber einem kleineren kann die Röntgenleistung gesteigert und damit die Scandauer verringert werden, gleichzeitig können durch das erfindungsgemäße Kippen Verluste an Bildschärfe ausgeglichen werden. Eine alternative Kathode 9 ist in FIG 11 gezeigt. Nach einer Ausgestaltung der Erfindung weist die Kathode 9 mindestens einen Emitter 14 mit mindestens einem Zwischenabgriff 15 zur separaten Ansteuerung von Teilbereichen des Emitters 14 auf; damit wird eine Auswahl zwischen mindestens zwei unterschiedlich großen Brennflecken ermöglicht, zum Beispiel derart dass statt eines Teilbereiches der gesamte Emitter 14 zur Erzeugung des Elektronenstrahles und damit des Brennfleckes verwendet wird. Dadurch ergibt sich wiederum eine Steigerung der Röntgenleistung und letztendlich eine Verkürzung der Scanzeit.

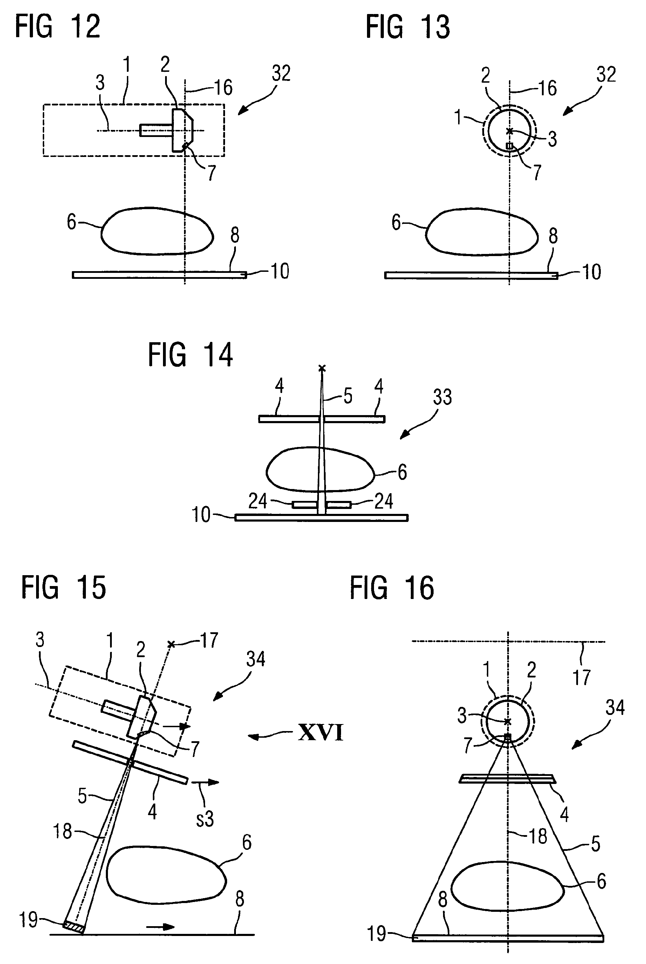

In FIG 12 bis 13 ist eine weitere Ausgestaltung der erfindungsgemäßen Röntgenvorrichtung 32 gezeigt. Zusätzlich zur erfindungsgemäßen Kippung ist die Röntgenröhre 1 in vorteilhafter Weise um eine erste, senkrecht zu dem Untersuchungsbereich 8 durch den Brennfleck 7 verlaufende Achse 16 drehbar. FIG 12 zeigt die Röntgenröhre 1 in einer ersten Position, zum Beispiel vor der Drehung um die erste Achse 16, FIG 13 zeigt dieselbe Röntgenröhre 1 nach einer solchen Drehung um 90°. Durch die Drehung erübrigt sich zum Beispiel eine Umpositionierung des Objektes 6. Besonders für einen Patienten wird dadurch die Untersuchung erleichtert und weniger unangenehm.

FIG 14 zeigt eine alternative Methode zur Reduzierung der Streustrahlung bei einer erfindungsgemäßen Röntgenvorrichtung 33. In zweckmäßiger Weise ist eine weitere bewegbare schlitzförmige Blende 24 vorgesehen, die detektornah angeordnet ist. Die detektornahe Blende 24 ist dazu derartig synchron zum Röntgenstrahlenbündel 5 über den Untersuchungsbereich 8 bewegbar, dass sie Streustrahlung ausfiltert. Die detektornahe Blende 24 kann alternativ als Streustrahlenraster ausgebildet sein und aus parallel zur Bewegungsrichtung des Röntgenstrahlenbündels angeordneten, kippbaren Lamellen bestehen. Die in FIG 12 und 13 gezeigte Drehung um die erste Achse 16 ermöglicht bei Nutzung der detektornahen Blende 24, dass die Öffnung der detektornahen Blende 24 bis direkt an den Rand des Untersuchungsbereiches 8 bewegbar ist; dies wurde bisher beispielsweise bei der Mammographie durch das Brustbein des Patienten verhindert.

Im Scanmodus ist es im Allgemeinen notwendig, auch die Blende um die erste Achse 16 zu drehen. Es kann auch der Detektor 10 mitgedreht werden. Zweckmäßigerweise ist ein Detektor 10 als eine den gesamten Untersuchungsbereich abdeckende Detektorfläche ausgebildet.

In für eine weitere Reduzierung einer Streustrahlung vorteilhafter Weise ist bei einer erfindungsgemäßen Röntgenvorrichtung 34 mit einem Detektor 19 zur Umwandlung von Röntgenstrahlung in ein Bildsignal der Detektor 19 als eine jeweils nur einen Teil des Untersuchungsbereiches abdeckende, sich quer zur Bewegungsrichtung des Röntgenstrahlenbündels erstreckende Detektorzeile 19 - wie unter anderem in FIG 15 und 16 gezeigt - ausgebildet und über den Untersuchungsbereich 8 bewegbar.

FIG 15 und FIG 16 zeigen außerdem in zwei verschiedenen Ansichten eine weitere Drehverstellmöglichkeit der Röntgenröhre 1. Die kippbare Röntgenröhre 1 ist zusätzlich in vorteilhafter Weise um eine zweite Achse 17 drehbar, die in der Ebene des fächerförmigen Röntgenstrahlenbündels 5, senkrecht zu der Drehachse 3 der Drehanode 2 und außerhalb des Brennfleckes 7 auf dessen dem Untersuchungsbereich 8 abgewandter Seite liegt. Durch diese Drehung um die zweite Achse 17 ist das fächerförmige Röntgenstrahlenbündel derart bewegbar, dass die Drehachse 3 der Drehanode 2 im Wesentlichen in der Ebene senkrecht zur Fächerebene des fächerförmigen Röntgenstrahlenbündels liegt. Zweckmäßigerweise sind der Brennfleck 7, der von der schlitzförmigen Blende 4 erzeugte Schlitz mit seinem Mittelpunkt und der Detektor 19 in einer Linie 18 anordbar und entlang dieser Linie 18 bewegbar. Werden zum Beispiel Röntgenröhre 1 und schlitzförmige Blende gemeinsam auf den Untersuchungsbereich 8 zubewegt, hat das den Vorteil, dass keine Verzerrungen der Abbildung auftreten können. Durch die Annäherung der Röntgenröhre an den Untersuchungsbereich kann die Röntgenleistung pro Fläche gesteigert und damit die Scanzeit verringert werden. Durch Drehung der Röntgenröhre um die zweite Achse 17 und gleichzeitige Anordnung von Röntgenröhre 1, schlitzförmiger Blende 4 und Detektorzeile 19 in einer Linie 18 können die schlitzförmige Blende 4 und die Detektorzeile 19 synchron zur Röntgenröhre 1 um die zweite Achse 17 gedreht werden und dadurch kann ebenfalls der Untersuchungsbereich 8 gescannt werden.

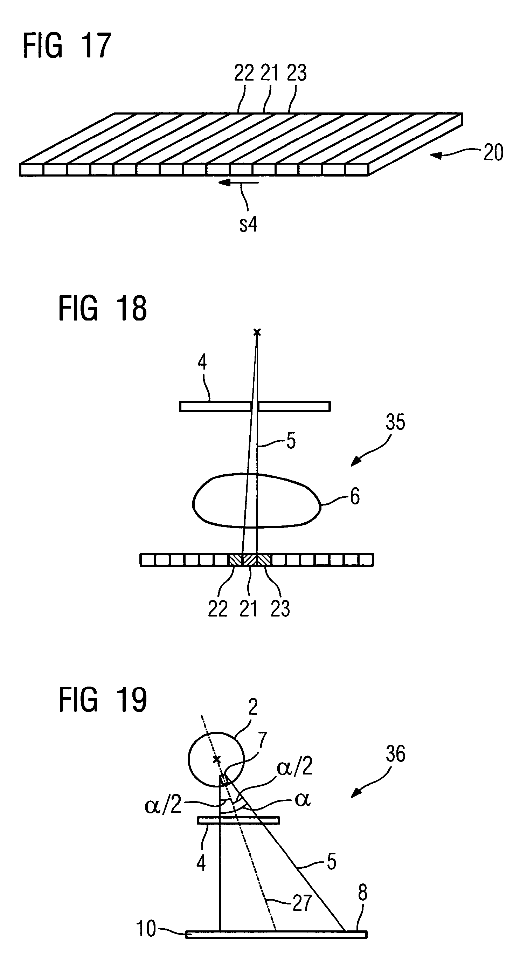

FIG 17 zeigt eine Ausgestaltung mit einem Detektor 20, der in vorteilhafter Weise aus einer Reihe von parallelen, senkrecht zur - durch den Pfeil s4 angedeuteten - Bewegungsrichtung des Röntgenstrahlenbündels 5 angeordneten Detektorzeilen, zum Beispiel 21-23, besteht.

Zur Vermeidung von Streustrahlung sind nach einer Ausgestaltung der Erfindung die Detektorzeilen jeweils in Abhängigkeit von einer Überstreichung durch das Röntgenstrahlenbündel 5 aktivierbar, wie in FIG 18 anhand der vom Röntgenstrahlbündel 5 überstrichenen Detektorzeile 21 gezeigt. In Erkenntnis, dass abhängig von dem abzubildenden Objekt auch die Streustrahlung wichtige Abbildungsinformationen, zum Beispiel für die Differentialdiagnostik, hervorbringen kann, sind in vorteilhafter Weise bei einer erfindungsgemäßen Röntgenvorrichtung 35 Detektorzeilen 22; 23 in Abhängigkeit von einer Überstreichung angrenzender Detektorzeilen 21 durch das Röntgenstrahlenbündel 5 zur Messung von Streustrahlung aktivierbar. Gemäß einer Ausgestaltung der Erfindung ist eine Schlitzweitenverstellung der schlitzförmigen Blende 4 in Steuerungsabhängigkeit von einer Veränderung des Verhältnisses der Streustrahlung zur Nutzstrahlung des Strahlenbündels im Sinne einer Verbreiterung der schlitzförmigen Blende 4 bei Verringerung des Verhältnisses und im Sinne einer Verengung der schlitzförmigen Blende 4 bei Vergrößerung des Verhältnisses vorgesehen. Bei sehr schmalem Schlitz ist zwar an sich die Streustrahlung gering, die Dauer des Scannens aber sehr hoch, was eine unerwünschte Belastung für den Patienten bedeuten kann.

Gemäß einer weiteren Ausgestaltung ist bei der erfindungsgemäßen Röntgenvorrichtung mit einer von einem Röhrenstrom abhängigen Dosis der Röntgenröhre eine Steuerung für die Dosis über den Röhrenstrom der Röntgenröhre mit einer Steuerungsabhängigkeit von einem Abbildungskontrast und einer Strahlungsbelastung eines Patienten im Sinne einer Erhöhung der Röntgendosis bei geringem Abbildungskontrast und im Sinne einer Erniedrigung der Röntgendosis bei hoher Strahlungsbelastung des Patienten vorgesehen. Durch die Einstellung der Dosis im zu Beginn des Scannens überstrichenen Bereich wird für den übrigen Bereich eine unnötige Strahlenbelastung eines Patienten vermieden und gleichzeitig der Kontrast der Röntgenabbildungen optimiert.

FIG 19 zeigt eine weitere vorteilhafte Ausgestaltung einer erfindungsgemäßen Röntgenvorrichtung 36. In zweckmäßiger Weise liegt die Verlängerung der radialen Erstreckung des Brennfleckes 7 in der Winkelhalbierenden 27 des Öffnungswinkels α des Röntgenstrahlenbündels 5. Dadurch wird zusätzlich die Bildschärfe der Röntgenabbildung gesteigert, da diese in der Ebene der Drehanode auf der Verlängerung des Brennfleckes am höchsten ist und bei Entfernung von dieser Linie geringer wird.

Die Erfindung lässt sich wie folgt kurz zusammenfassen: Zur Verbesserung der Bildauflösung bzw. Bildschärfe ist bei einer Röntgenvorrichtung mit einer Röntgenröhre zur Erzeugung eines von einem Brennfleck einer um eine Drehachse drehbaren Drehanode ausgehenden Röntgenstrahles und mit einer schlitzförmigen Blende zur Erzeugung eines aus dem Röntgenstrahl einblendbaren, scannerartig über einen Untersuchungsbereich bewegbaren, fächerförmigen Strahlenbündels vorgesehen, dass das fächerförmige Strahlenbündel im wesentlichen in Richtung der Drehachse der Drehanode über den Untersuchungsbereich bewegbar ist und die Röntgenröhre derart um den Brennfleck kippbar ist, dass das fächerförmige Röntgenstrahlenbündel bei der Bewegung über den Untersuchungsbereich im Bereich höchster Bildauflösung liegt.

## Patentansprüche

1. Röntgenvorrichtung (31-36), insbesondere für ein Mammographie-Röntgengerät,
- mit einer Röntgenröhre (1) zur Erzeugung eines von einem Brennfleck (7) einer um eine Drehachse (3) drehbaren Drehanode (2) ausgehenden Röntgenstrahles,
- mit einer schlitzförmigen Blende (4) zur Erzeugung eines aus dem Röntgenstrahl einblendbaren, scannerartig über einen Untersuchungsbereich (8) bewegbaren, fächerförmigen Röntgenstrahlenbündels (5),
wobei
- das fächerförmige Röntgenstrahlenbündel (5) durch Kippen der Röntgenröhre (1) um den Brennfleck (7) im wesentlichen in Richtung der Drehachse (3) der Drehanode (2) über den Untersuchungsbereich (8) bewegbar ist,
- die Röntgenröhre (1) derart um den Brennfleck (7) kippbar ist, dass das fächerförmige Röntgenstrahlenbündel (5) bei der Bewegung über den Untersuchungsbereich (8) im Bereich höchster Bildauflösung bzw. höchster Bildschärfe liegt.

2. Röntgenvorrichtung nach Anspruch 1, wobei
die schlitzförmige Blende (4) im Wesentlichen in Richtung der Drehachse (3) der Drehanode (2) und im Wesentlichen synchron zur Bewegung des fächerförmigen Röntgenstrahlbündels (5) über den Untersuchungsbereich (8) bewegbar ist.

3. Röntgenvorrichtung nach Anspruch 1, wobei
die schlitzförmige Blende (4) im Sinne eines den gesamten Untersuchungsbereich (8) überdeckenden Schlitzes verstellbar ausgebildet ist.

4. Röntgenvorrichtung nach Anspruch 1, wobei
die schlitzförmige Blende (4) im Sinne eines den gesamten Untersuchungsbereich (8) überdeckenden Schlitzes verstellbar ausgebildet und das Röntgenstrahlenbündel (5) mit seinem Bereich höchster Bildauflösung innerhalb dieses Schlitzes durch das Kippen der Röntgenröhre (1) auf einen definierten Bereich des Untersuchungsbereichs (8) einstellbar ist.

5. Röntgenvorrichtung nach einem der vorangehenden Ansprüche, wobei der Brennfleck (8) in radialer Richtung der Drehanode (2) vergrößerbar ist.

6. Röntgenvorrichtung nach Anspruch 5 mit einer Kathode (9) zur Erzeugung eines Elektronenstrahls, wobei die Kathode (9) mindestens zwei Emitter (12; 13), insbesondere unterschiedlicher Ausprägung zur Erzeugung eines Brennfleckes (7) entsprechender Abmessungen, aufweist.

7. Röntgenvorrichtung nach Anspruch 5 und/oder 6 mit einer Kathode (9) zur Erzeugung eines Elektronenstrahls, wobei die Kathode (9) mindestens einen Emitter (14) mit mindestens einem Zwischenabgriff (15) zur separaten Ansteuerung von Teilbereichen des Emitters (14) aufweist.

8. Röntgenvorrichtung nach einem der vorangehenden Ansprüche, wobei die Röntgenröhre (1) zusätzlich um eine erste, senkrecht zu dem Untersuchungsbereich durch den Brennfleck verlaufende Achse (16) drehbar ist.

9. Röntgenvorrichtung nach einem der vorangehenden Ansprüche, wobei die kippbare Röntgenröhre zusätzlich um eine zweite Achse (17) drehbar ist, die in der Ebene des fächerförmigen Röntgenstrahlenbündels (5), senkrecht zu der Drehachse (3) der Drehanode (2) und außerhalb des Brennfleckes (7) auf dessen dem Untersuchungsbereich (8) abgewandter Seite liegt.

10. Röntgenvorrichtung nach einem der Ansprüche 1 bis 9 mit einem Detektor (10; 20) zur Umwandlung von Röntgenstrahlung in ein Bildsignal, wobei der Detektor als eine den gesamten Untersuchungsbereich abdeckende Detektorfläche ausgebildet ist.

11. Röntgenvorrichtung nach einem der Ansprüche 1 bis 9 mit einem Detektor (19) zur Umwandlung von Röntgenstrahlung in ein Bildsignal, wobei der Detektor (19) als eine jeweils nur einen Teil des Untersuchungsbereiches abdeckende, sich quer zur Bewegungsrichtung des Röntgenstrahlenbündels erstreckende Detektorzeile (19) ausgebildet und über den Untersuchungsbereich (8) bewegbar ist.

12. Röntgenvorrichtung nach Anspruch 9 und 11, wobei der Brennfleck (7), der Schlitz mit seinem Mittelpunkt und der Detektor (19) mit seinem Mittelpunkt in einer Linie (18) anordbar und entlang dieser Linie (18) bewegbar sind.

13. Röntgenvorrichtung nach einem der vorangehenden Ansprüche, wobei mindestens eine weitere bewegbare schlitzförmige Blende (24) vorgesehen ist, die detektornah angeordnet ist.

14. Röntgenvorrichtung nach Anspruch 10, wobei der Detektor (20) aus einer Reihe von parallelen, senkrecht zu der Bewegungsrichtung des Röntgenstrahlenbündels angeordneten Detektorzeilen (21-23) besteht.

15. Röntgenvorrichtung nach Anspruch 14, wobei die Detektorzeilen (21-23) jeweils in Abhängigkeit von einer Überstreichung durch das Röntgenstrahlenbündel (5) aktivierbar sind.

16. Röntgenvorrichtung nach einem der Ansprüche 14 und/ oder 15, wobei Detektorzeilen (21-23) in Abhängigkeit von einer Überstreichung angrenzender Detektorzeilen (21-23) durch das Röntgenstrahlenbündel (5) zur Messung von Streustrahlung aktivierbar sind.

17. Röntgenvorrichtung nach einem der vorangehenden Ansprüche, wobei die erste schlitzförmige Blende (4) zwei im Sinne einer Schlitzweitenverstellung gegeneinander und/oder zueinander bewegbare, den Schlitz begrenzende Blendenbacken (4.1; 4.2) aufweist.

18. Röntgenvorrichtung nach Anspruch 15 bis 17, wobei die Schlitzweitenverstellung der schlitzförmigen Blende (4) in Steuerungsabhängigkeit von einer Veränderung des Verhältnisses von Streustrahlung zu Nutzstrahlung des Röntgenstrahlenbündels im Sinne einer Verbreiterung der schlitzförmigen Blende (4) bei Verringerung des Verhältnisses und im Sinne einer Verengung der schlitzförmigen Blende (4) bei Vergrößerung des Verhältnisses gestellt ist.

19. Röntgenvorrichtung nach Anspruch 15 mit einer von einem Röhrenstrom abhängigen Dosis der Röntgenröhre, wobei
eine Steuerung für die Dosis über den Röhrenstrom der Röntgenröhre (1) mit einer Steuerungsabhängigkeit von einem Abbildungskontrast und einer Strahlungsbelastung eines Patienten im Sinne einer Erhöhung der Röntgendosis bei geringem Abbildungskontrast und im Sinne einer Erniedrigung der Röntgendosis bei hoher Strahlungsbelastung des Patienten vorgesehen ist.

20. Röntgenvorrichtung nach einem der vorangehenden Ansprüche, wobei die radiale Erstreckung des Brennfleckes (7) auf der Winkelhalbierenden (27) des Öffnungswinkels (α) des Röntgenstrahlenbündels (5) liegt.

21. Mammographie-Röntgengerät mit einer Röntgenvorrichtung nach zumindest einem der Ansprüche 1-20.
